(19) Europäisches Patentamt
European Patent Office
Office européen des brevets

(11) Publication number: 0 435 509 A2

# (12) EUROPEAN PATENT APPLICATION

(21) Application number: 90313490.6

(22) Date of filing: 12.12.90

(51) Int. Cl.⁵: H01L 31/113, A61B 6/00

(30) Priority: 26.12.89 US 456902

(43) Date of publication of application:
03.07.91 Bulletin 91/27

(84) Designated Contracting States:
DE FR GB NL

(71) Applicant: GENERAL ELECTRIC COMPANY
1 River Road
Schenectady, NY 12345(US)

(72) Inventor: Brown, Dale Marius
2338 St Joseph Drive
Schenectady, New York 12309(US)
Inventor: Michon, Gerald John
24 Broad Street
Waterford, New York 12188(US)

(74) Representative: Pratt, Richard Wilson et al
London Patent Operation G.E. TECHNICAL
SERVICES CO. INC. Burdett House 15/16
Buckingham Street
London WC2N 6DU(GB)

(54) Semiconductor photodetector and method of operation.

(57) The photodetector device (10) has a large detection area in combination with a low output capacitance. A photodetector system employing this device can provide low noise as a result of the device's low output capacitance. The device (10) preferably comprises a body (12) of semiconductor material of one conductivity type with a large detector region (22) of the opposite conductivity type disposed adjacent a major surface and with a substantially smaller output region (26) also of the second conductivity type disposed adjacent the same surface and spaced from the detector region. The detector region (22) is coupled to the output region (26) by an insulated gate electrode (34) which controls the potential underneath the gate electrode. The output region (26) is biased to reverse bias its PN junction with the body region and the gate electrode (34) is biased in the same polarity with a magnitude to maintain the channel region at a lesser potential so that the floating detector region is at a lesser potential than the output region and capacitively decoupled from the output region.

## SEMICONDUCTOR PHOTODETECTOR AND METHOD OF OPERATION

The present invention relates to semiconductor photodetectors and to a method of operating such a photodetector.

Semiconductor devices, particularly diodes, have long been used to sense the presence of light and to operate systems in accordance with the presence or absence of light. In many such systems, the photodetector serves as an on/off actuator and is mounted in a circuit which is neither designed to nor capable of providing any measurement of the light input other than whether or not the light input exceeds a threshold level which controls circuit switching.

In other systems, it is desired to measure the light intensity which is incident on the photodetecting diode. One such system is a computed tomography (CT) x-ray scanning system which employs a solid, luminescent scintillator to convert incident x-rays to luminescent light. In such CT scanning systems, a multicellular x-ray scintillation detector system may comprise 1,000 or more individual cells, each cell including a separate scintillator block and a photoresponsive diode to convert the scintillator's light output to an electrical signal. Such scanning systems include a gantry on which an x-ray source is mounted on one side of a measurement zone and an x-ray scintillation detector system is mounted on the opposite side of the measurement zone in alignment with the x-ray beam. Such systems preferably employ a fan-shaped x-ray beam and a scintillation x-ray detector which may comprise 1,000 or more separate detection cells.

For maximum data collection accuracy, the individual cells of the scintillator detection system are disposed immediately adjacent to each other, thereby maximizing the collection by the scintillation detector system of the x-rays emerging from the patient or other object being examined. The individual blocks of scintillator material may typically be about 3 mm deep in a direction parallel to the x-ray beam propagation direction, by about 1 mm wide in the lengthwise direction of the scintillation detector array, by about 30 mm long in the direction perpendicular to the plane of the x-ray fan beam. The ~3 mm depth of the scintillator block is determined in accordance with the x-ray stopping power of that block and the percentage of the x-rays it is desired to stop within the scintillator block. The ~1 mm width of the scintillator block in the lengthwise direction of the detector array is determined in accordance with the desired data resolution along the length of the detector array. The ~30 mm length of the scintillator of the block perpendicular to the plane of the x-ray fan beam is determined by the thickness of the x-ray fan beam in combination with the desired vertical thickness of the measurement zone. Naturally, other factors also are involved in these choices.

For ease of manufacture and assembly, it is preferred to integrate a number of the photoresponsive diodes in a single chip or wafer of semiconductor material. A corresponding number of the scintillator blocks are then assembled with such a chip to form a module containing that number of scintillation detector cells. In this module, the patterned side of the semiconductor structure and the electrodes thereon face the blocks of scintillator material for maximum collection of luminescent light. The immediate side-by-side placement of adjacent scintillation detector cells requires that the output connections to the individual diodes be located adjacent a narrow end of the diode in order that wires, conductors and other optical obstructions may be excluded from the active collection area of the diode to maximize light collection, system efficiency and cell-to-cell uniformity.

To generate a computed tomography image, the gantry is rotated about the measurement zone while the x-ray beam is on and the output from the scintillation detector system is recorded or stored for concurrent or subsequent processing. Data is taken continuously during each revolution of the gantry. At each position where data is taken, the output of each of the detector cells is determined and stored.

In such a system, the vast quantity of data which has been recorded ($^{\sim}4 \times 10^5$ data points) is subsequently processed to generate an image of the object or patient disposed in the measurement zone during the measurement process.

Each data point comprises the position of the detection cell at the time the data was taken and the amplitude of the output from the photodiode associated with that cell at that time. All of these output amplitudes are processed using computerized tomography image reconstruction techniques, which are known in the art, to generate an image of the object in the measurement zone. The accuracy of the generated image depends on the accuracy with which incident x-ray intensity on the scintillator cell is converted to an electronic signal having an amplitude which is a measure of that x-ray intensity.

Thus, image accuracy is limited by the ability of the scintillation x-ray detection system to accurately convert x-ray intensity to electronic signal amplitude. In order to maximize throughput, minimize x-ray exposure and patient inconvenience, it is considered desirable to perform a data collection scan as rapidly as possible. In order to provide accurate image generation with low intensity x-rays and high scan rates, it is necessary that the photodetection system accurately transduce incident luminescent light intensity to a corresponding electrical signal amplitude in a linear manner across a wide dynamic range including both very low and very high light intensities, a dynamic range of $10^4$ to $10^5$ or more being considered desirable.

Because of this wide desired dynamic range and the desire to be able to operate at very small rates of photo generated charge carriers, it is necessary to minimize noise contributions to signals throughout the overall data acquisition system.

Present General Electric Company CT scanners of the Zeus type employ a solid ceramic scintillator to convert incident x-ray intensity to luminescent light. A large area PIN photosensitive diode on the order of 3/4 mm x 30 mm in area is optically coupled to the scintillator block and detects the luminescent light produced thereby and converts that light to an output current whose amplitude is linearly related to the intensity of the luminescent light and thus to the incident x-ray intensity, provided the scintillator provides a linear x-ray-to-luminescent-light conversion. This output current is in turn converted to an output voltage by an operational amplifier which is dedicated to that photosensitive diode.

Many design considerations go into the design of the photodetector diode and its operational amplifier output system. For accurate image generation, each of the scintillator detector cells must provide substantially identical conversion of x-ray intensity to electronic signal amplitude. Consequently, it is necessary to minimize offset voltages among the operational amplifiers connected to the various photodetector diodes. This is facilitated by operating the diode in a zero bias (photoconductive) mode to minimize offset voltages and thermal drift.

Such large photoconductive diodes, even with a zero bias depletion depth on the order of 10 microns, have a capacitance of about 300 pf or about 13 pf/mm$^2$. As is well known in the operational amplifier art, the noise in an operational amplifier is directly proportional to the capacitance at its input node. One way of improving the signal-to-noise ratio of the overall detector system is to reduce the capacitance at the input of the operational amplifier by reducing the capacitance of the photodetector diode. The output capacitance of the photodetector diode could be reduced to about 30 pf by reverse biasing the diode at about 100 volts. However, this would increase the reverse bias dark current considerably and increase the noise caused by "shot" noise fluctuations in this current. In addition the operational amplifier offset voltages would be large. Further, such a large reverse bias is inconsistent with the input requirements of the semiconductor operational amplifiers which serve as the output amplifiers. Consequently, some other means of reducing system noise is needed.

Since the capacitance of a diode is proportional to its area, shrinking the diodes could reduce system electrical noise. However, since the size of the scintillator blocks is dictated by the x-ray stopping power, beam pattern and required resolution requirements of the system, an optical system would be needed to concentrate the scintillator light on a diode which was sufficiently small to improve the noise performance significantly. That optical system would itself degrade overall system performance too much for such a means of reducing noise to be feasible.

A wide variety of photodetector focal plane arrays are known in the art. These include CCD, CID and similar many-cell-by-many-cell detector arrays. Such imagers are normally built with as small a pixel or cell size as possible in order to facilitate high resolution transduction of the image. Such focal plane arrays are employed to detect and transduce an image which is focused on the detector array. As such, they may be read out at a frame rate which is consistent with the information rate present in the image to be detected and the rate at which meaningful data can be absorbed by the intended use system. Charge coupled device (CCD) imagers employ surface potentials to form isolated detector potential wells or cells within a continuous region of uniform semiconductor structure. CCD systems are run in a clocked manner in which (1) the image is integrated for a period of time by storing photogenerated charge within the individual potential wells of the photodetecting array and (2) that integrated image is then read out rapidly and the integration of another image is started. In order to control charge transfer through a CCD imager structure, transparent insulated gate electrodes must be disposed over the CCD structure to impose appropriate potentials on the various segments of the semiconductor structure to form the isolated potential wells during integration and to cause transfer of the resulting charge packets through the structure during the readout portion of the process.

A CCD imager is not practical as the luminescent light sensor in a CT scanning system because an image is not being sensed and each scintillation detector cell must have a large photocollection area. Such a large area CCD would add unnecessary complications and cost to the system without any corresponding benefit, since the CCD structure is designed for high resolution, real time, image conversion which is not needed. Further more, the yield of CCD with the large size ($~$3 x 0.1 cm²) required for each cell would be low, which would lead to excessive costs.

Embodiments of the invention, as disclosed herein, seek to provide:

a very large area, low capacitance semiconductor photodetector device suitable for use in photodetection systems;

such a device which has a simple, easily and inexpensively manufactured structure;

a means of operating a measuring photodetection system to produce stringent linearity and minimum noise in order to provide maximum image resolution in a CT scanning system;

a semiconductor photodetector whose output capacitance is substantially independent of the size of its photodetection area; and/or

a diode-like photodetector with an output which is capacitively decoupled from the collector area.

In accordance with one embodiment of the the present invention a photodetector device has a body region of one type conductivity, and separate, spaced apart detector and output regions of opposite type conductivity disposed therein and forming individual PN junctions therewith. The body and detector regions have separate electrodes in ohmic contact therewith. The detector and output regions are capacitively decoupled by using an insulated gate electrode which controls the potential in a channel region extending between the detector and output regions. In operation, the device is biased with the output/body PN junction at a relatively high reverse bias with the detector region floating at a potential which reverse biases the detector/body PN junction. The potential of the floating detector region is controlled by the bias applied to the gate electrode. The device is preferably operated with a fixed gate voltage to provide a continuous current output.

So long as a potential difference is maintained between the detector and output regions, the output capacitance of the device is controlled by the size and structure of the output region and its overlap with the gate electrode, independent of the size of the detector region.

A highly linear, minimum noise data acquisition system results from the low capacitance of the output region. The absence of gate electrodes disposed over the detector region maximizes quantum efficiency.

In accordance with an alternative embodiment of the invention, the insulated gate electrode is made a resistive gate which produces a potential gradient in the channel region to assist charge carrier transport from the detector region to the output region. This may preferably be accomplished by use of a resistive material for the gate electrode and by disposing high conductivity, parallel to the length of the gate, conductors in ohmic contact therewith at the two ends of the channel region. These two conductors are held at different potentials to create a parallel-to-the-length of the channel potential gradient to cause the enhanced carrier transport.

In accordance with another alternative embodiment, the opposite conductivity type doping in the detector region is omitted, and a resistive gate structure is disposed across the entire photocollection area. This resistive gate extends all the way to the output region. Once again, high conductivity electrodes are disposed in ohmic contact with the resistive gate layer at opposite ends of its "channel length" to induce a potential gradient which assists all collected charges in transport from their location of collection into the output region for readout. This latter embodiment provides a linear light-in-to-signal-out conversion characteristic at lower photocarrier generation rates than does the initially described embodiment.

The invention, both as to organization and method of practice, both as to organization and method of practice, may be better understood by reference to the following illustrative description taken in connection with the accompanying drawings in which:

Figure 1 illustrates in schematic form a portion of a computed tomography machine employing the present invention;

Figure 2 is a schematic, perspective illustration of a semiconductor photoresponsive device in accordance with the present invention;

Figure 3 is a cross-section of the Figure 2 structure taken along the line 3-3 in Figure 2;

Figures 4A-4C are aligned with Figure 3 and illustrate potentials in the structure of Figure 3 under different bias conditions;

Figures 5-8 illustrate alternative plan views for the structure of Figure 2;

Figures 9, 12 and 15 illustrate alternative device structures; and

Figures 10, 11; 13, 14 and 16 and 17 illustrate the structures and potentials in the devices of Figures 9, 12 and 15, respectively.

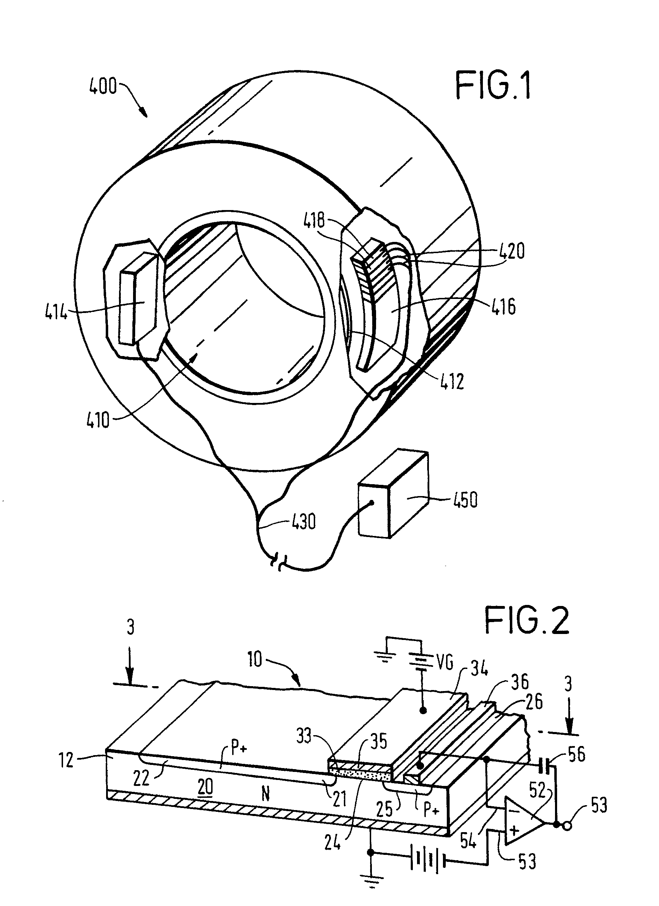

A computed tomography (CT) scanning system 400 is illustrated schematically in Figure 1. This CT scanning system 400 comprises a cylindrical enclosure 410 in which the patient or object to be scanned or examined is positioned. A gantry 412 surrounds the cylinder 410 and is configured for rotation about the cylinder concentric with the cylinder's axis. The gantry 412 may be designed to revolve for one full revolution and then return or may be designed for continuous rotation, depending on the system used to connect the electronics on the gantry to the rest of the system. The electronics on the gantry include an x-ray source 414 which produces a fan x-ray beam which encompasses a scintillation detector system 416 mounted on the gantry on the opposite side of the cylinder 410. The fan pattern of the x-ray source is disposed in the plane defined by the x-ray source and the scintillation detector system 416. The scintillation detector system 416 is very narrow or thin in the direction perpendicular to the plane of the x-ray fan-beam. Each cell 418 of the scintillation detector system 416 incorporates a solid transparent bar of scintillator material and a photodetector diode optically coupled to that scintillator bar. The output from each photodetector diode is connected to an operational amplifier. The output from each operational amplifier is connected either by individual wires 420 or by other electronics to the main control system 450 for the computed tomography system. In the illustrated embodiment, power for the x-ray source and signals from the scintillation detector are carried to the main control system 450 by a cable 430. The use of the cable 430 generally limits the gantry to a single full revolution before returning to its original position. Alternatively, slip rings or optical or radio transmission may be used to connect the gantry electronics to the main control system 450 where continuous rotation of the gantry is desired. In CT scanning systems of this type, the scintillator material is used to convert incident x-rays to luminescent light which is detected by the photodetector diode and thereby converted to an electrical signal as a means of converting the incident x-rays to electrical signals which may be processed for image extraction and other purposes.

In Figure 2, a portion of a device 10 in accordance with the present invention is shown in a perspective, cross-sectional view. The device 10 comprises a body 12 of semiconductor material, including an N type body region 20, a P+ detector region 22 which extends into the body region 20 and forms a first PN junction 21 with that region, an output P+ region 26 which extends into the body region 20 and forms a second PN junction 25 therewith and an insulated gate electrode 34 disposed over the body region between its detector and output regions. The detector region 22 and the output region 26 are spaced apart in the X-direction in the figure by a channel portion 24 of the body region 20. A body region electrode 30 is disposed in ohmic contact with the back surface of the body region 20 and extends over substantially all of that back surface of the semiconductor body 12. An output region electrode 36 is disposed in ohmic contact with the output region 26 and extends along the length thereof to minimize the output resistance within the output region 26. The insulated gate electrode 34 is comprised of an insulating layer 33 and a conducting layer 35, is disposed on the upper surface of the semiconductor body 12 and extends from adjacent to or over detector region 22 to adjacent to or over the output region 26 to control the potential and conductivity therebetween of the channel region portion 24 of the body region 20. The conducting layer 35 may be metal, heavily doped polysilicon, silicide or other high conductivity material. The purpose of the conducting layer 35 being to impose a substantially uniform potential on the entire channel region. For operation, a bias and output circuit 50 is connected to the body region electrode 30, the output region electrode 36 and the gate electrode 34. The bias and output circuit 50 includes an operational amplifier 52 having non-inverting and inverting input terminals 53 and 54, respectively, an output terminal 55 and a feedback or integrating capacitor 56. The inverting input terminal 54 of the operational amplifier is connected to the output electrode 36 of the device. The non-inverting input terminal 53 of the operational amplifier is connected to a voltage source which has an output voltage $V_D$ which is negative. The ground terminal of the voltage source is connected to the body region electrode 30. The gate electrode 34 is connected to ground through a second voltage source which provides an output voltage $V_G$ which is also negative. For proper operation of this device and bias/output circuit in the preferred mode of operation, the magnitude of $V_G$ is less than the magnitude of $V_D$.

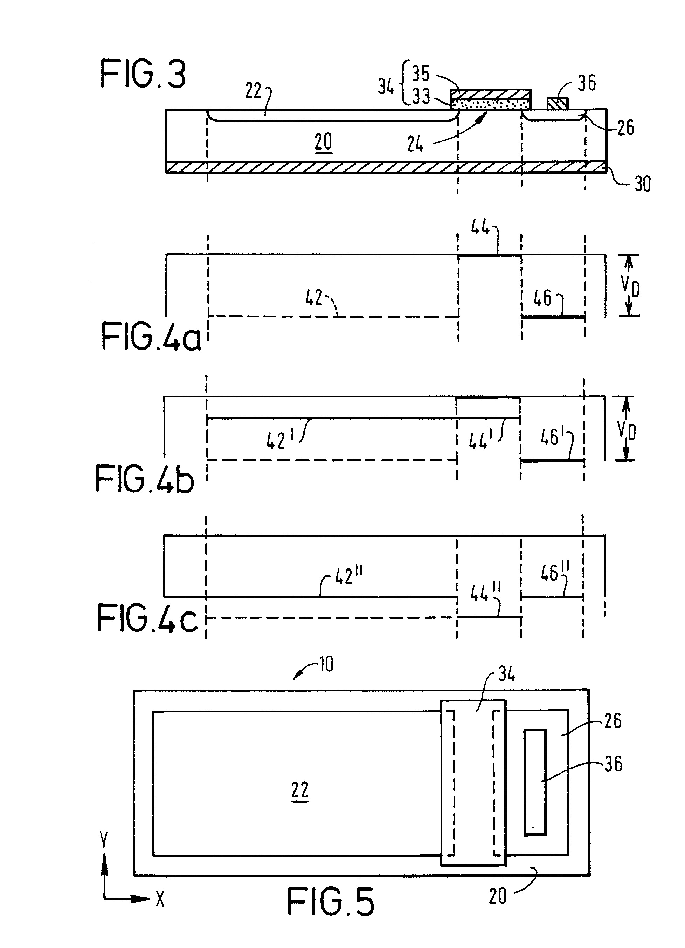

Figure 3 is a cross-section through Figure 2 along the lines 3-3 for purposes of providing orientation and alignment for Figures 3A-3C to be discussed below. In Figure 3, the device 10 has its detector region 22 and its output region 26 both rendered as rectangles having vertical sides with the gate electrode 34 overlapping both of these regions. The dotted lines extending from Figures 3 through Figures 4A-4C are for purposes of alignment of the Figure 4 illustrations with the Figure 3 structure.

In Figure 4A, a negative bias $V_D$ is applied to the output region electrode 36. As a result, the potential in the output region 26 is substantially below ground and constitutes a potential well identified by the reference numeral 46 in Figure 4A. Thus, the PN junction 25 is reverse biased. In Figure 4A, the gate electrode 34 is maintained at ground potential or at a positive potential relative to ground. Consequently, the potential in the channel region 24 of the body region 20 is at or above ground potential as indicated by the reference numeral 44. Since there is no ohmic connection to the detector region 22 in the structure 10 of Figures 1 and 2 the potential of the detector region 22 is undetermined in Figure 4 since that region is floating. As indicated by the dotted line 42 in Figure 4A, the potential in the detector region may be below ground.

In Figure 4B, the potentials for the structure of Figure 3 are illustrated under modified (preferred operating) bias conditions. The bias voltage applied to the output electrode 36 is still $-V_D$ with the result that the potential well 46' in the output region has the same depth as in Figure 4A. However, for the Figure 4B illustration, the gate electrode 34 is biased at a negative voltage which is between ground and the voltage $V_D$. Consequently, the potential in the channel region portion 24 of the body region 20 is below ground as is illustrated by the reference numeral 44'. The detector region 22 under these bias conditions, is held at a potential which is no greater than the potential in the channel region as illustrated by reference numeral 42', since any charge carriers at a higher potential than 44' will diffuse through the channel region 24 into the output region 26. The potential 44' can be referred to as a shelf potential, since that potential controls the potential in the photo charge accumulation region at that "shelf potential". The output region's lower potential 46' prevents diffusion out of the output region into the channel and detector regions. Thus, the potential in the detector region will be at the same level as the potential 44'. Consequently, the PN junction 21 is reverse biased. Because of the reverse bias on the PN junction 21, any photo-induced positive charge carriers (holes) in the body region 20 in the vicinity of the region 22 will be attracted across the PN junction 21 into the detector region 22. Such charges will tend to raise the potential of the region 22 above the level 44'. Under those conditions, the collected charges flow through the channel region as a diffusion current because the lower potential 46' in the output region results in one way transport of holes under the gate electrode from the detector region into the output region and not from the output region to the detector region. These holes, as collected in the output region, flow into the output circuit as an electron current in a well known manner. The greater the incident light intensity on the detector region 22, the more intensive is the generation of photo-induced hole-electron pairs with a resultant increase in current flow under the gate electrode into the output region. Consequently, the output of the operational amplifier 52 is proportional to the intensity of light striking the detector region. Because the potential well 46' in the output region is deeper than the potential well 42' in the detector region, the detector region potential well 42' is capacitively decoupled from the output region-potential well 46'. That is, since (1) holes cannot flow from the output region 26 into the channel region 24 or the detector region 22, and (2) the flow of holes into the output region 22 is independent of the potential of that region (as long as the output region potential is lower than the channel region potential, in both cases), changes in output region potential affect charge storage only in the output region itself. As a consequence, only the output potential well 46' contributes to the output capacitance of the photodetector device. Consequently, the gate electrode 34 in combination with the structure of the photocharge accumulation region and the output region serves as a capacitance decoupler and the output capacitance of this device under these operating conditions is substantially independent of the size of the photodetector region 22. Thus, the objective of providing a photodetector device whose output capacitance is substantially independent of the photocollection area has been achieved.

In Figure 4C, the potentials in the device are shown for a further modified bias condition which is not considered desirable in the preferred method of operating the device. In the Figure 4C illustration, the potential applied to the gate electrode 34 is more negative than the potential applied to the output electrode 36. As a consequence, the potential well 44" under the gate electrode is deeper than the potential well under the drain electrode and will fill to the level of the output region's potential. As a consequence, the detector region potential well 42" is directly connected to the drain region potential well 46" with the result that there is no capacitive decoupling between these two potential wells and the output capacitance of the device is a result of the combined areas of the output, channel and detector regions. Such a mode of operation is not preferred where a low output capacitance is desired or required.

For high incident light intensities, many photo-induced holes are generated and the current flow under the gate electrode is substantial under the Figure 4B bias conditions. Under these conditions, the output of the operational amplifier 52 is a linear function of the incident light intensity. As the incident light intensity decreases, current flow through the channel region decreases. At a sufficiently low incident light intensity, current flow in the channel region is so low that the channel is in the subthreshold region of the insulated gate (MOSFET) conduction characteristics. In this region the channel mobility of charge carriers is very low and the time required to allow the charge transfer from the collector area through the channel to the output node becomes very long. Thus, for short measurement intervals, the device becomes nonlinear because charge generated in the extended area diode region can not be transferred rapidly enough to the output node. The light intensity to which linearity is maintained in this device decreases as the resistivity level of the body region 20 increases. Consequently, the resistivity of the body region is one of the design factors which can be adjusted to reduce the light level at which non-linearity sets in in this device. The problem of subthreshold operation can be alleviated by increasing the current level so as to "raise" the channel characteristic out of the subthreshold region. This is sometimes called the use of a "fat" zero condition and can be achieved by illuminating the collection area with a steady bias light intensity or by increasing the device temperature to increase its dark current. However, both of these solutions will increase noise. For many system applications of a device of this type, either the small non-linearity effect (parts per million range) or increased noise would not be a problem. However, where maximum image resolution, minimum noise and maximum linearity are desired in a CT scanning system, the resulting non-linearity of output versus incident light intensity for a low level of incident light can be a limiting factor in system operation.

The structure of device 10 is simple to build and should be fabricatable with high yield since it is essentially two diodes integrated in a single wafer with a single insulated gate disposed thereon. The device may be fabricated with a reasonable channel length which assures high yield in fabrication. Channel lengths in a range from 2 microns to 20 microns are acceptable. It will be recognized that the greater the channel length, the higher the light intensity at which non-linearity in operational amplifier output begins to become noticeable as the light intensity decreases.

If desired, the operational amplifier 52 may be integrated on the same wafer with the photodetector device. Further, as has been discussed above, it is preferred in systems such as CT scanning systems, to integrate these photodetector devices for several different scintillator cells in a single semiconductor wafer to simplify manufacturing and packaging of the overall scintillation detector system.

Figures 5-8 illustrate, in plan view, alternative structures for the device 10. In Figure 5, the device 10 is illustrated with the output region 26 and the channel region 24 having the same Y-direction extent as the detector region 22.

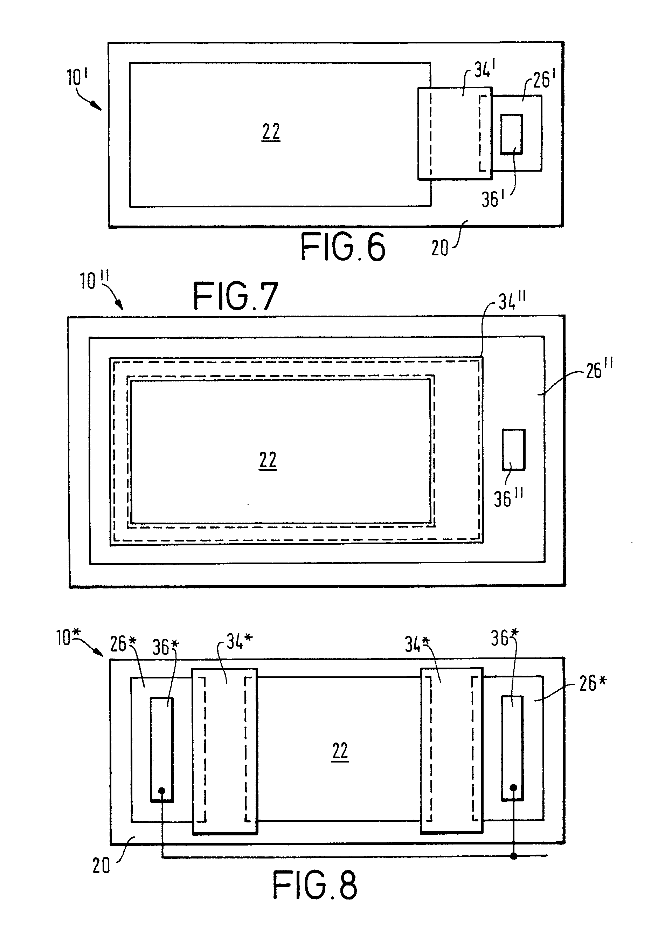

In Figure 6, the device 10' has an output region 26' and a channel region 24', both of which are smaller in the Y-direction than the detector region 22. This structure has the advantage of increasing the current flow through the channel region for low light intensities. This reduces the light intensity threshold at which non-linearity reaches any given level.

In Figure 7, the device 10" has been modified by providing an output region 26" which surrounds the detector region 22. The channel region 24" is a similar rectangular annulus extending from the detector region 22 to the output region 26". This structure reduces the current density in the channel region and can be desirable for systems intended for use with high light intensities, but has the disadvantage for systems for use with low light intensity of increasing the threshold level of light intensity at which non-linearity in the operational amplifier output sets in.

A further alternative structure 10* is illustrated in plan view in Figure 8. The Figure 8 structure is similar to the Figure 5 structure, except for the addition of a second output region 26*, a second output electrode 36* and a second gate electrode 34* with a corresponding channel 24* at the left-hand end of the device structure. This structure may be considered desirable where the X-direction extent of the detector region 22 is substantial compared to the diffusion length of holes in the detector region 22.

While it is possible, in principle, to determine and use an output intensity correction table which corrects for the non-linearity of the output for low light intensity, that adds to system complication and is not a preferred solution. The light intensity level at which this non-linearity occurs may be substantially reduced by providing a potential gradient under the gate electrode which slopes from the detector region toward the output region in a manner to provide a potential-gradient-induced drift velocity for carriers flowing through the channel region. Where the output potential is -5 volts, a potential difference of as little as 2 volts from the left-hand side of the gate electrode to the right-hand side of the gate electrode can reduce the light intensity at which excessive non-linearity occurs.

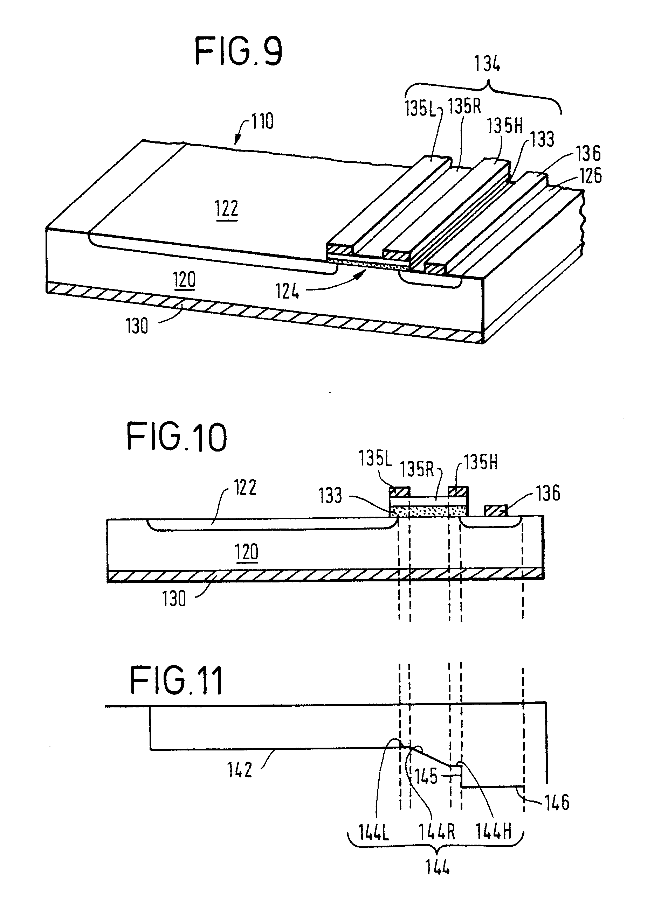

Such a potential gradient is provided by the alternative embodiment of the present invention which is illustrated generally at 110 in Figure 9. In this embodiment, rather than a uniform highly conducting layer 35 being used as the conducting portion of the gate electrode 34, as illustrated in Figure 2, the device 110 incorporates a resistive layer 135R as the conductive portion of the gate electrode. This resistive layer may be polysilicon which is appropriately doped to provide the desired resistivity. the purpose of the polysilicon layer being to enable a potential gradient to be imposed across the channel region from the photocharge accumulation region to the output region. This resistive layer is provided with two separate metal contacts or highly conductive electrode segments 135L and 135H which extend perpendicular to the length of the channel region. Separate bias potentials are applied to the two metal contacts 135L and 135H. In particular, contact 135H has a higher potential magnitude applied to it than is applied to contact 135L. As a result of these differing potentials, current flows between contacts 135L and 135H through the resistive portion 135R, thereby developing a resistive voltage drop which creates the desired slope to the potential under the gate electrode. This establishes a potential gradient in the channel region which adds a drift component to the charge carrier flow through the channel region. This results in the desired current linearity at substantially lower incident light levels than is provided by the device 10 of Figure 2.

Each of the elements of device 110 in Figure 9 is identified by a reference numeral which is larger by 100 than the corresponding structure in the device 10 of Figure 2. Each of these elements serves the same purpose and functions in a similar manner as the corresponding elements in Figure 2 in a manner which will be well understood by those skilled in the art, with the exception of those elements which have been discussed here. The structure of device 110 of Figure 9 is significantly more complicated to fabricate than the device structure 10 of Figure 2. Consequently, the device structure 10 will be preferred for those applications where extreme linearity (parts per million) for extremely low light intensities is not required. On the other hand, the device structure 110 of Figure 9 (and modifications thereof) will be preferred for those situations in which extreme linearity for extremely low light intensities is required.

Figures 10 and 11 respectively illustrate the structure of the device 110 of Figure 9 in cross-section in a manner similar to the Figure 3 illustration of the device 10 structure and the potentials present in the structure of Figure 10 under conditions similar to those present in Figure 4B with respect to device 10.

In Figure 11, the output region 126 has a potential well 146 therein as a result of the bias applied to the output electrode 136. In the channel region, the potential well 144 has three different segments to its bottom. Adjacent the output region 126, the potential well has a flat bottom 144H. Adjacent the detector region 122, the potential well has a flat region 144L. Intermediate these two flat portions, a sloped portion 144R extends between and connects these two flat portions as a result of the presence of the resistive layer 135R in the gate conductor 135. The depth or level of the flat portion 144H is determined by the potential applied to the contact 135H of the gate conductor 135. In a similar manner, the level of the potential 144L is determined by the potential applied to the contact 135L of the gate conductor 135. The sloping portion 144R of the potential merely connects the levels 144L and 144H in accordance with the current flow in and the voltage drop across the resistive layer 135R between the contacts 135L and 135H. The height of the step 145 between the potential 144H under the right-hand edge of the gate electrode and potential well 146 in the output region is the difference in the potentials applied to the gate contact 135H and the output electrode 136. The potential 142 in the detector region 122 is the same as the level 144L established by the potential applied to the contact 135L of the gate conductor 135. Each of these various potential levels is independently controllable by the bias voltages applied to the three conductors 135L, 135H and 136.

It is preferred to operate the device with a sufficient potential difference applied to the contact 135H with respect to the potential applied to the electrode 136 to ensure that the step in potential 245 is greater than any variation in the potential at the electrode 136 to ensure that only the output region 126 and any gate electrode overlap contribute to the output capacitance of the device.

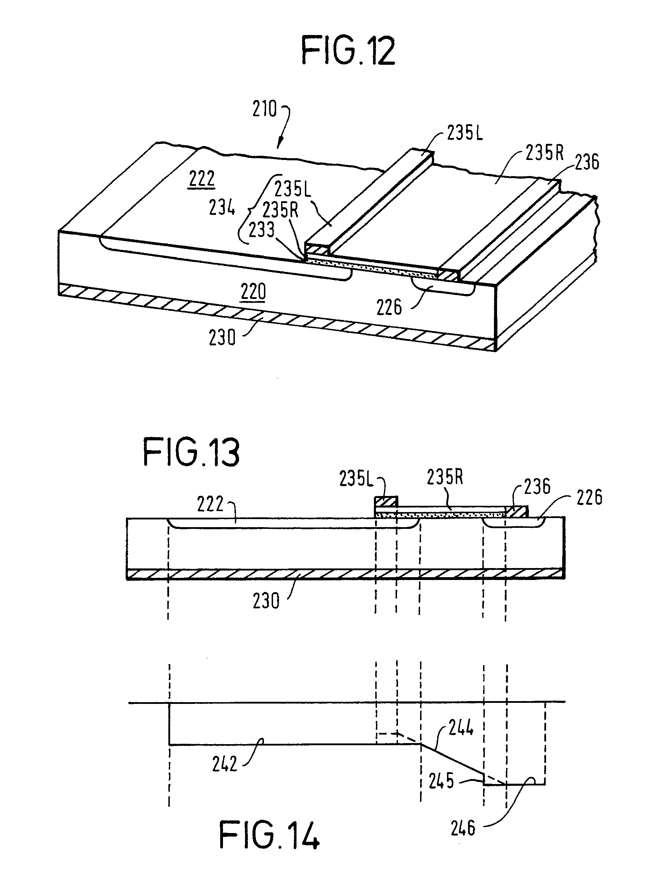

An alternative version 210 of the device 110 is illustrated in a perspective cross-section view in Figure 12. The device 210 is similar to the device 110 except for the configuration of its gate electrode 234. The insulating layer 233 of the gate electrode 234 extends substantially further over the detector region 222 and the output region 226 than is the case with the insulating layer 133 of the insulated gate in device 110. Further, the resistive layer 235R of the gate electrode in device 210 extends substantially the full width of the insulating layer 233. At the detector region side of the gate electrode, a metal contact 235L of the gate electrode conductor 235 is disposed in ohmic contact with the resistive layer 235R portion of the conductive portion 235 of the gate electrode 234. This configuration is similar to that in the device 110 of Figure 9. However, in the device 210, the gate conductor 235 has no separate metal contact corresponding to contact 135H of the gate conductor in the device 110. Instead, the right-hand edge, in the figure, of the resistive layer 235R is disposed in ohmic contact with the output region electrode 236 of the device 210. This gate electrode structure has the advantage that only a single gate potential is required to operate the device and that gate potential is applied to the contact 235L of the gate electrode. This contrasts with the structure of the device 110 where separate gate biases must be applied to the two metal contacts 135L and 135H of the gate conductor 135. In the device 210, rather than providing a second gate bias voltage, the bias voltage applied to the output region via the electrode 236 serves as that second gate bias voltage. Thus, the device 210 of Figure 12 requires only the same three external connections as the device 10 of Figure 2, rather than requiring four separate external connections as is the case with the device 110 of Figure 9. It will be recognized that the device structure of Figure 9 could be provided with only three external connections by internally connecting the contact 135H to the output region electrode 136.

In Figure 13, the device structure of Figure 12 is illustrated in cross-section view similar to the view in Figure 10 of the structure of Figure 9. In Figure 13, the potentials are illustrated for a condition similar to that in Figure 11. In particular, with a bias voltage applied to the output electrode 236 which places the second PN junction 235 in substantial reverse bias, a potential well having a depth 246 is created in the output region. In the gate region 224, the potential 244 slopes from the value 242 in the detector region to the edge of the potential well 246 in the output region 226. The height of the step at the edge of the potential well 246 with the channel region slope 244 is determined by the threshold voltage of the MOS gate.

So long as the step in potential 245 at the edge of the output region is greater than the fluctuations in potential on the output electrode 236, the channel and extended area diode regions are decoupled from the output and do not contribute to the output capacitance of the device.

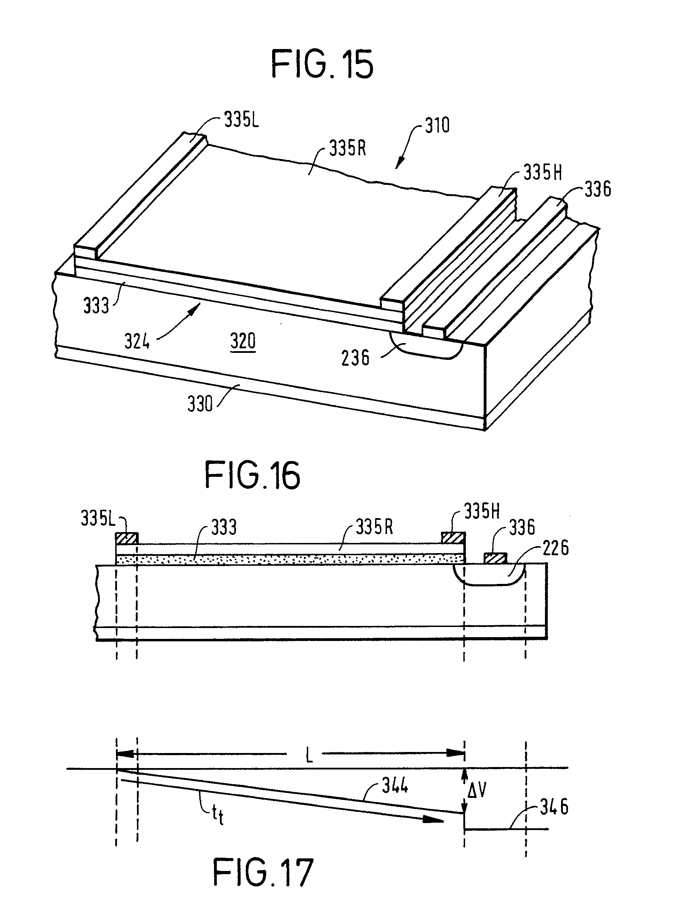

A further alternative embodiment 310 of a device in accordance with the present invention is illustrated in a perspective, cross-section view in Figure 15. This structure is similar to the structure 110 except that (1) the resistive gate layer 335R extends across the entire light collection region with its contacts 335L and 335H disposed at opposite ends of that resistive gate layers channel length and (2) in the absence of a detector region which is impurity doped to a conductivity type opposite to that of the body region 320. In this device, the resistive gate layer 335R and the gate insulating layer 333 need to be made transparent in order that the light to be detected can pass therethrough to generate hole/electron pairs within the body 320 of the semiconductor material. Consequently, the resistive gate layer 335R is preferably a thin, appropriately doped polysilicon layer which may preferably be about 500Å thick. The structure of device 310 is shown in Figure 16 in a cross-section similar to Figures 3, 10 and 13. The potentials induced in the structure during operation are illustrated in Figure 17 in a manner similar to Figures 4, 11 and 14. The potential in the output region is the lowest potential (greatest magnitude) in the structure as indicated by the line 346. The potential in the photoresponsive or detector region is sloped downward toward the output region as indicated by the line 344. Where the light collection region has a length L, and the potential in the detection region at the edge of the output region is lower by $\Delta V$ than the potential at the opposite edge of the collection region, the transit time $t_t$ is given by:

$$t_t = \frac{L^2}{\mu \Delta V},$$

where L is the length of the collection region channel, $\Delta V$ is the potential difference and $\mu$ is the charge carrier mobility.

For a device having a detector region which is 30 mm long, $L^2$ is substantial. Since output linearity is dependent on the transit time, as the length L increases, the potential difference $\Delta V$ must also increase to keep linearity constant. However, the depth of the output region potential well must be kept at a level which is compatible with the semiconductor operational amplifiers which convert the current from the output region into a voltage.

For a device structure of this type, the maximum charge carrier transit time can be reduced by 75% by placing the output region at the center of the detector region. In this manner, each half of the collection region has a length L/2 which leads to a factor of 4 reduction in the transit time since L is squared in the transit time equation.

The length L of the channel in the collector region of a long, narrow photodetector of this type can be minimized by placing the output region along a long side rather than a short side of the detector region. For a 30 mm x 1 mm collector region, this reduces L by a factor of 30 and the transit time by a factor of 900, thereby substantially reducing the light level at which non-linearity becomes a problem.

In all of the embodiments of this invention, in order to maintain linearity to as low a light intensity as possible, it is preferred to construct the channel regions as buried channel regions rather than surface channel regions in order to avoid charge trapping in fast interface states which can affect charge transfer. Such buried channels may be fabricated in a manner well known in the semiconductor art.

While the contacts '35L and '35H have been described as being metal contacts, it will be understood by those skilled in the art that metal silicides and other highly conductive, non-metal contacts may be used.

As has been discussed, the CT solid luminescent scintillator structure constrains its associated photodetector to have a large collection area. It is not feasible to focus such a large collector area onto a significantly smaller detector because presently available optical reduction systems lose too much light in the reduction process for effective use with the low light levels present at the low end of the dynamic range of such a system. The present invention solves the problem of linear photodetection in an extreme situation in which very low light levels must be detected in a system having a dynamic range of $10^5$ with a structure having a low output capacitance for noise reduction purposes where the photodetector has a very large photo/charge conversion area, all with linearity in the parts per million range. Thus, this invention has operating characteristics which are way beyond anything obtainable with prior art photodetectors.

While the invention has been described in detail herein in accord with certain preferred embodiments thereof, many modifications and changes therein may be effected by those skilled in the art. Accordingly, it is intended by the appended claims to cover all such modifications and changes as fall within the true spirit and scope of the invention.

## Claims

1. A photosensitive semiconductor device comprising:
   a body of one type conductivity; a photo charge accumulation region of opposite type conductivity forming a first PN junction with said body;
   an output region of said opposite type conductivity, spaced from said photo charge accumulation region and forming a second PN junction with said body;
   a channel region portion of said body extending from said photo charge accumulation region to said output region; and
   a MOS gate disposed adjacent said body for controlling the potential of said photo charge accumulation region by controlling the potential of said channel region portion of said body and thus the potential a charge carrier of said opposite type conductivity must have in order to flow from said photo charge accumulation region through said channel region to said output region.

2. The photosensitive semiconductor device recited in claim 1 wherein:
   said photo charge accumulation region has an area at least ten times the area of said output region.

3. The photosensitive semiconductor device recited in claim 1 wherein:
   said photo charge accumulation region has an area of at least 20 mm2.

4. The photosensitive semiconductor device recited in claim 3 wherein:
   said device, when operated with a fixed gate bias which renders said channel region conductive to charge carriers of said opposite type conductivity, has an output capacitance as measured at said output region of less than 50 pf.

5. The photosensitive semiconductor device recited in claim 1 wherein:
   said device, when operated with a fixed gate bias which renders said channel region conductive to charge carriers of said opposite type conductivity, has an output capacitance as measured at said output region of less than 2.5 pf/mm2.

6. A circuit comprising:
   a photosensitive semiconductor device of the type including a body region of one type conductivity, a photo charge accumulation region of opposite type conductivity forming a first PN junction with said body region, an output region of said opposite type conductivity, spaced from said photo charge accumulation region and forming a second PN junction with said body region, and a MOS gate electrode disposed adjacent said body region for controlling the conductivity of a channel region portion of said body region for charge carriers of said opposite conductivity type, said channel region portion of said body region extending from said photo charge accumulation region to said output region;
   means for biasing said output region to reverse bias said second PN junction;
   means for applying a bias voltage to said MOS gate electrode which renders said channel region conductive to carriers of said opposite type conductivity of more than a shelf potential, whereby said first PN junction becomes reverse biased; and
   means for sinking current from said output region and providing an output signal representative of the amplitude of the current sunk from said output region.

7. The circuit recited in claim 6 wherein:
   said output current is at least in part photo-induced current; and
   said output signal is representative of the intensity of light incident on said device.

8. The circuit recited in claim 6 wherein: the amplitude of said output signal is representative of the intensity of said incident light.

9. The circuit recited in claim 6 further comprising:
   means for periodically sampling said output signal to sense the present light intensity.

10. The photosensitive semiconductor device recited in claim 6 wherein:
said MOS gate is configured to impose a potential gradient on said channel region to provide drift assisted carrier flow through said channel region.

11. The device recited in claim 1 or 10 wherein:
said device includes an output region electrode disposed in ohmic contact with said output region; and
said MOS gate is configured to impose a potential gradient on said channel region to provide drift-assisted carrier flow through said channel region.

12. The photosensitive semiconductor device recited in claim 23 wherein said gate includes:
a resistive layer which spans said channel region; first and second high conductivity electrode segments disposed in ohmic contact with said resistive layer and spaced apart in the direction of the channel region's length.

13. The photosensitive semiconductor device recited in claim 23 wherein said gate includes:
a layer of electrically resistive material extending across said channel region to said output region electrode; and
a highly conductive electrode segment disposed in ohmic contact with said resistive layer remote from said output region electrode.

14. The photosensitive semiconductor device recited in claim 13 wherein:
said layer of electrically resistive material is ohmically connected to said output region.

15. The photosensitive semiconductor device recited in claim 14 wherein:
said layer of electrically resistive material is disposed in ohmic contact with said output region electrode.

16. The photosensitive semiconductor device recited in any preceding claim wherein:
said photo charge accumulation region is free of ohmic contacts thereto.

17. The photosensitive semiconductor device recited in any preceding claim further comprising an output region electrode disposed in ohmic contact with said output region.

18. A method of operating a photosensitive semiconductor device of the type including a body region of one type conductivity, a photo charge accumulation region of opposite type conductivity forming a first PN junction with said body region, an output region of said opposite type conductivity, spaced from said photo charge accumulation region and forming a second PN junction with said body region, and a channel region portion of said body region extending from said photo charge accumulation region to said output region, a MOS gate electrode disposed adjacent said body for controlling the potential of said photo charge accumulation region by controlling the potential of said channel region portion of said body and thus the potential a charge carrier of said opposite type conductivity must have in order to flow from said photo charge accumulation region through said channel region to said output region, said method comprising:
biasing said output region to reverse bias said second PN junction; and
applying a steady gate bias potential to said MOS gate electrode which renders said channel region conductive to carriers of said opposite type conductivity of more than the desired potential for said photo charge accumulation region, whereby said first PN junction becomes reverse biased to attract photo induced opposite type conductivity charge carriers from said body region into said photo charge accumulation region.

19. The method recited in claim 16 wherein: said gate bias potential induces a potential gradient in said channel to assist charge carrier flow from said detector region to said output region.

20. A semiconductor photodetector comprising: a detector diode;
an output diode; and
a capacitance decoupler for controlling conduction between said detector diode and said output diode while decoupling the capacitance of said output diode from the capacitance of said detector diode.

21. The semiconductor photodetector recited in claim 30 wherein:
said detector diode, said output diode and said capacitance decoupler are integrated on a single body of semiconductor material.

22. The semiconductor photodetector recited in claim 31 wherein:
said capacitance decoupler comprises an insulated gate electrode which is disposed on said body of semiconductor material and which extends from a first region of said detector diode to the first region of said output diode to control the conductivity of a channel region extending from said first region of said detector diode to said first region of output diode.

23. The semiconductor photodetector recited in claim 22 wherein:
said body of semiconductor material comprises a body region of one type conductivity;
said detector diode comprises a detector region of opposite type conductivity forming a first PN junction with said body region;
said output diode comprises an output region of opposite type conductivity forming a second PN junction with said body region.

24. The semiconductor photodetector recited in claim 23 wherein:
said capacitance decoupler comprises an insulated gate electrode which is disposed on said body of semiconductor material over a portion of said body region and which extends from said detector region to said output region to control the conductivity of a channel region which extends from said detector region to said output region.

25. The semiconductor photodetector recited in claim 22 wherein:
said first PN junction has an area which is at least five times the area of said second PN junction.

26. The semiconductor photodetector recited in claim 25 wherein:
said first PN junction has an area which is at least ten times the area of said second PN junction.

27. The semiconductor photodetector recited in claim 26 wherein:
said first PN junction has an area which is at least fifty times the area of said second PN junction.

28. The semiconductor photodetector recited in claim 37 wherein:
said first PN junction has an area which is at least one hundred times the area of said second PN junction.

29. A photodetector system comprising:
a body of semiconductor material including a body region of one type conductivity;
a detector region and an output region, each of opposite type conductivity, each disposed in said body of semiconductor material and, respectively, forming first and second PN junctions with said body region;
a capacitance decoupler for controlling conduction between said detector region and said output region while decoupling the capacitance of said output region from the capacitance of said detector region, said decoupler comprising an insulated gate electrode disposed on said body of semiconductor material and extending from said detector region to said output region to control the conductivity of a channel region extending from said detector region to said output region for opposite conductivity type charge carriers;
means for biasing said output region relative to said body region in a manner to reverse bias said second PN junction and for biasing said capacitance decoupler in a manner to maintain the channel region conductive to carriers of said opposite type conductivity of a potential to maintain said second PN junction less reverse biased than said second PN junction whereby charge carriers of said opposite type conductivity flow from said detector region to said output region, but not from said output region to said detector region.

30. The photodetector system recited in claim 29 further comprising:
signal processing means connected to said output region for collecting current from said output region.

31. The photodetector system recited in claim 39 wherein:
said capacitance decoupler is configured to induce a potential gradient in said channel region in the direction from said detector region to said output region.

32. A photosensitive semiconductor device comprising:
a body of one type conductivity;
a photo charge accumulation region portion of said body, said photo charge accumulation region being of one type conductivity;
an output region of said opposite type conductivity forming a PN junction with said body;
a resistive MOS gate disposed adjacent said body for imposing a potential gradient on said photo charge accumulation region to provide drift field assisted charge carrier transport across said photo charge accumulation region toward said output region.

33. The photosensitive semiconductor device recited in claim 32 wherein:
said photo charge accumulation region has an area at least ten times the area of said output region.

34. The photosensitive semiconductor device recited in claim 32 wherein:
said photo charge accumulation region has an area of at least 20 mm2.

35. The photosensitive semiconductor device recited in claim 34 wherein:
said device, exhibiting an output capacitance as measured at said output region of less than 50 pf.

36. The photosensitive semiconductor device recited in claim 32 wherein:
said resistive gate is substantially transparent.

37. The photosensitive semiconductor device recited in claim 32 wherein said resistive gate includes:
a layer of electrically resistive material extending across said photo charge accumulation region to said output region electrode; and
a highly conductive electrode segment disposed in ohmic contact with said resistive layer remote from said output region electrode.

38. The photosensitive semiconductor device recited in claim 32 further comprising:
an output region electrode disposed in ohmic contact with said output region.

FIG.1

FIG.2

FIG.3

FIG.4a

FIG.4b

FIG.4c

FIG.5

15

FIG.6

FIG.7

FIG.8

# FIG.9

# FIG.10

# FIG.11

# FIG.12

210

222
234
235L
235R
233
235L
235R
236

220

226

230

# FIG.13

222
235L
235R
236
226

230

# FIG.14

242
244
245
246

# FIG.15

# FIG.16

# FIG.17